(19) Europäisches Patentamt
European Patent Office
Office européen des brevets

(11) Publication number: **0 249 224 A2**

# (12) EUROPEAN PATENT APPLICATION

(21) Application number: **87108411.7**

(22) Date of filing: **11.06.87**

(51) Int. Cl.[4]: **C07K 5/06**, A61K 37/02, A61K 37/64

(30) Priority: **13.06.86 US 873939**

(43) Date of publication of application: **16.12.87 Bulletin 87/51**

(84) Designated Contracting States: **AT BE CH DE ES FR GB GR IT LI LU NL SE**

(71) Applicant: **MERRELL DOW PHARMACEUTICALS INC.**
**2110 E. Galbraith Road**
**Cincinnati Ohio 45215(US)**

(72) Inventor: **Flynn, Gary A.**
**7121 Euclid Drive**
**Cincinnati Ohio 45243(US)**
Inventor: **Beight, Douglas W.**
**645 Glensprings Drive**
**Cincinnati Ohio 45246(US)**

(74) Representative: **Macchetta, Francesco et al**
**Gruppo Lepetit S.p.A. Patent and Trademark Department 34, Via Roberto Lepetit**
**I-21040 Gerenzano (Varese)(IT)**

## (54) Novel antihypertensive agent.

(57) This invention relates to derivatives of fused cyclic azepin-2-ones, to the intermediates and processes useful for their preparation, and to their use as angiotensin converting enzyme inhibitors and to their end-use application as antihypertensive agents.



Xerox Copy Centre

# NOVEL ANTIHYPERTENSIVE AGENT

This invention relates to derivatives of fused cyclic azepin-2-ones, to the intermediates and processes useful for their preparation, and to their use as angiotensin converting enzyme inhibitors and to their end-use application as antihypertensive agents.

More specifically this invention relates to derivatives of fused cyclic azepin-2-ones of the formula

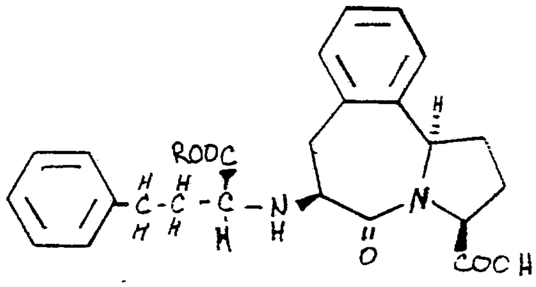

I

and the pharmaceutically acceptable salts thereof wherein R is $C_{1-6}$ lower alkyl, the most preferred of which is ethyl.

The compounds of formula I may be prepared by processes in the Reaction Scheme A using techniques well known in the art of peptide chemistry. The preferred diastereomeric forms are as depicted. Mixtures of these diastereomeric forms are comtemplated, as well as the individual and mixtures of the enantiomeric forms thereof.

REACTION SCHEME A

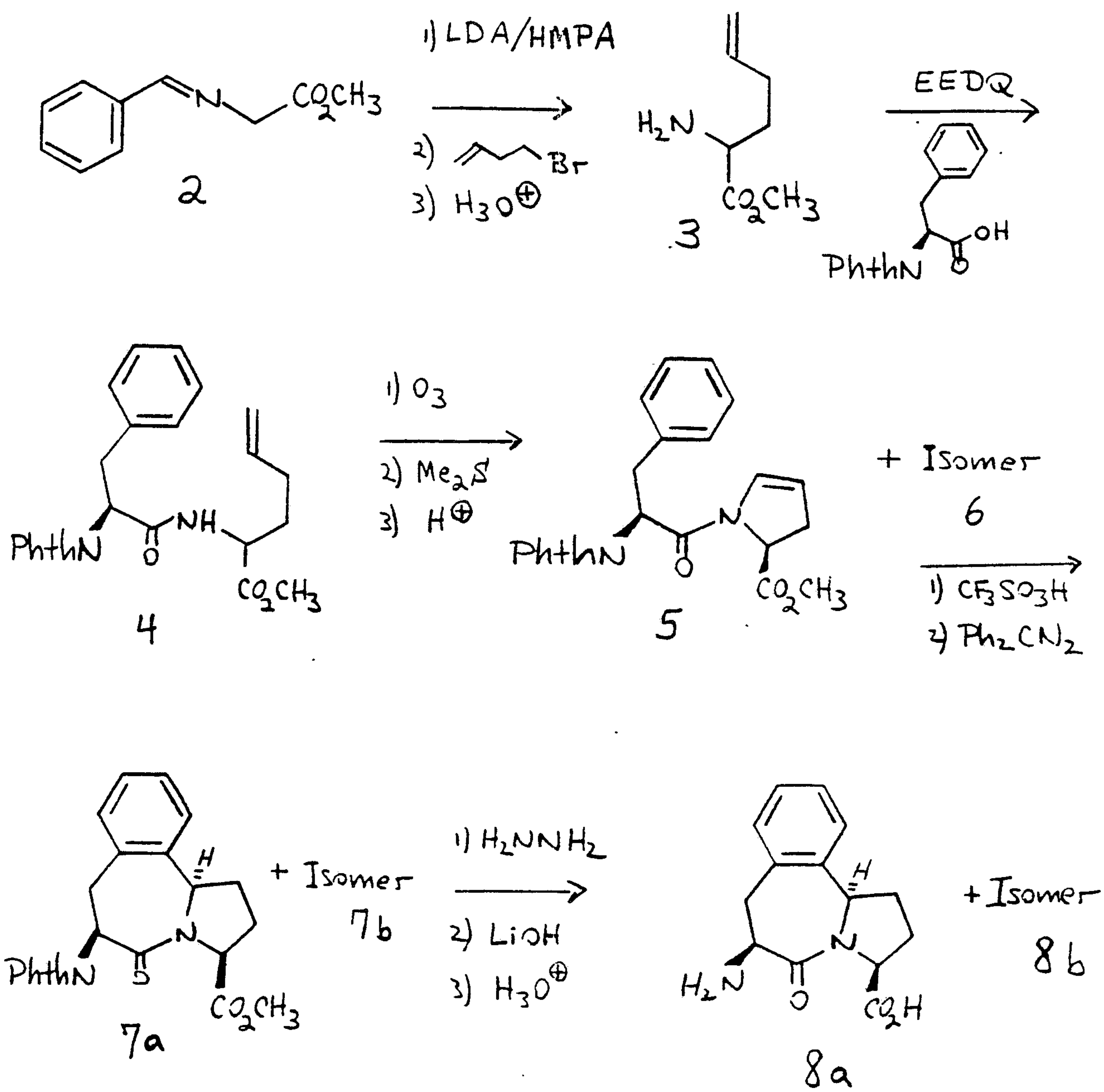

REACTION SCHEME A - Cont.

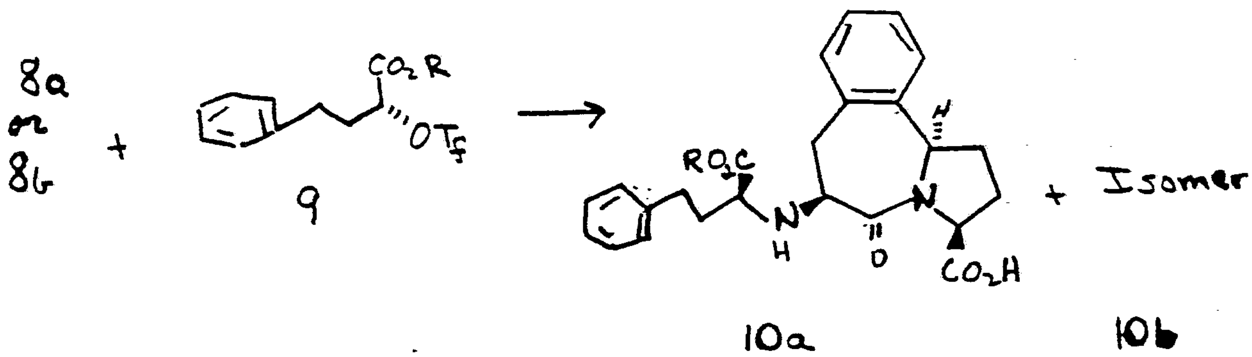

8a or 8b + 9 → 10a + Isomer 10b

wherein R is a $C_{1\text{-}6}$ lower alkyl, preferably ethyl and Phth together with the N atom represents a phthalimido protecting group (as shown in formula 4), LDA being lithium diisopropylamide, HMPA being hexamethylphosphoric triamide, and EEDQ being N-carbethoxy-2-ethoxy-1,2-dihydroquinoline and $T_f$ being trifluoromethanesulfonyl.

In general the foregoing reaction scheme starts with an appropriate Schiff base of methyl glycinate which is deprotonated with a strong base, e.g., LDA in THF and alkylated in the presence of HMPA with 4-bromo-1-butene, the intermediate product is hydrolized to its free amine by dilute mineral acid. The desired unsaturated alpha amino ester is coupled to N-phthaloyl-L-phenylalanine by the action of standard coupling reagents e.g., EEDQ. The resulting diastereomeric amides are treated with ozone in an inert solvent containing methanol at -70°C. Upon saturation the ozonide is reduced with dimethylsulfide and stablized with pyridine. The crude products are islolated and dehydrated by treatment with strong acid in an inert solvent yielding chromatographically separable acylenamines. These are then treated with trifluoromethanesulfonic acid to give a mixture of isomeric tricyclic lactams. The point of isomerism is about the carbon atom bearing the terminal carbomethoxy group. The individual isomeric compounds are deprotected by sequential treatment with hydrazine and lithium hydroxide. The desired zwitterion is coupled with the R-triflate (9) to give the requisite pro-drug monoester.

EXAMPLE 1

2-Amino-5-hexenoic acid, methyl ester

To a solution of 15.4 mL (110 mM) of diisopropylamine in 250 mL THF at -78°C was added 39 mL (105 mM) 2.7 M n-BuLi in hexane. After stirring for 30 min. a solution of 17.7 g (100 mM) of benzaldehyde-methyl glycinate Schiff base (1) in 25 mL THF was added over a 30 min. period. 20 mL HMPA was added followed by a solution of 13.5 g (100 mM) of 4-bromo-1-butene in 20 mL THF. The solution was warmed to 25°C and stirred for 3 hours. The reaction mixture was quenched into brine and extracted into ether. The organic layer was repeatedly washed with brine, dried over $MgSO_4$ and concentrated. The residue (25 g) was dissolved in 400 mL ether and stirred with 150 mL 1N HCl for 2 hours. The aqueous layer was removed and adjusted to pH=9. Extraction with $CHCl_3$ gave 8.5 g (60% yield) of a light oil; NMR ($CDCl_3$); 5.7 (m,1H); 5.0 (m,2H); 3.7 (s,3H); 3.42 (dd,1H,Ja=7$H_z$, Jb=6$H_z$); 1.6-2.3 (m,4H).

EXAMPLE 2

2-[[2(1,3-dihydro-1,3-dioxy-2H-isoindol-2-yl)-1-oxo-3-phenylpropyl]amino]-5-hexenoic acid, methyl ester

To a stirred solution of 6.0 g (20 mM) of N-phthaloyl-L-phenylalanine and 6.0 g (24 mM) of EEDQ in 30 mL dry $CH_2Cl_2$ at 25°C under $N_2$ atmosphere was added 3.0 g (21 mM) of methyl-2-amino-5-hexenoate in 5 mL $CH_2Cl_2$. After stirring for 18 hours, the solution was poured into 100 mL $CH_2Cl_2$, washed with 2 X 100 mL 10% HCl solution, saturated $NaHCO_3$ solution, dried over $MgSO_4$, and concentrated to give 8.3 g of a yellow oil. NMR ($CDCl_3$) 7.70 (m,4H); 7.10 (m,5H); 6.65 (d,1H,J=7$H_z$); 4.95 (m,3H); 5.41 (m,1H); 3.60 (s,3H); 3.45 (d,2H,J=7$H_z$; 1.7-2.3 (m,4H) was consistant with the diastereomeric amides.

EXAMPLE 3

2,3-dihydro-1-[2-(1,3-dihydro-1,3-dioxo-2H-isoindol-2-yl-1-oxo-3-phenylpropyl]-1H-pyrrole-2-carboxylic acid, methyl ester

A stirred solution of unsaturated amide (4) in 100 mL $CH_2Cl_2$ and 10 mL abs. methanol was cooled to -70°C and treated with a stream of ozone until a blue color persisted. The excess $O_3$ was removed with $N_2$ gas and the cooled reaction was treated with 5 mL $Me_2S$ and 0.5 mL pyridine. The solution was allowed to gradually warm to 25°C and stir for 18 hours. The solution was washed with 10% HCl solution, $H_2O$, and brine. The organic layer was dried over $MgSO_4$ and concentrated. The residue (4.5 g) was dissolved in 150 mL $CH_3CCl_3$ and treated with 0.5 mL trifluoroacetic acid at reflux for 18 hours. The solution was cooled, concentrated and flash chromatographed using 35-50% EtOAc/hexane. Chromatography gave 700 mg of a less polar acylenamine (Rf=0.70, 1:1 EtOAc/Hexane: NMR ($CDCl_3$); 7.70 (m,4H); 7.20 (m,5H); 6.50 (m,1H); 6.2-6.4 (m.2H); 4.8 (dd,1H, Ja=11$H_z$; Jb=6$H_z$; 3.76 (s,3H); 3.68 (dd,1H,Ja=17$H_z$,Jb=11$H_z$); 3.50 (dd,1H,Ja=17H $_z$,Jb=6$H_z$); 2.4-3.2 (m,2H) and 600 mg of a more polar acylenamine (Rf=0.62, 1:1 EtoAc/Hexane): NMR ($CDCl_3$); 7.70 (m,4H); 7.20 (m,5H); 6.55 (m,1H); 6.2-6.4 (m,2H); 4.9 (dd,1H,Ja=11 $H_z$,Jb=6$H_z$); 3.66 (S,3H); 3.64 (dd,1H,Ja=17$H_z$,Jb=11 $H_z$); 3.45 (dd,1H,Ja=17 $H_z$,Jb=6$H_z$); 2.3-3.3 (m,2H).

EXAMPLE 4

7-(1,3-dihydro-1,3-dioxo-2H-isoindol-2-yl-1,2,3,4,6,7,8,12b-octahydro-6-oxopyrido[2,1-a][2]benzazepine-4-carboxylic acid, methyl ester (mixture of isomers)

Both acylenamines(5) and (6) were cyclized separately. Less polar acylenamine (5) (700 mg) was dissolved in 5 mL dry $CH_2Cl_2$ and stirred with 2 mL $CF_3SO_3H$ at 25°C under $N_2$ for 18 hours. The reaction mixture was quenched with $H_2O$ and extracted into ethyl acetate. The organic extracts were washed well with $H_2O$, dried over $MgSO_4$ and treated with excess diazomethane. Flash chromatography gave 305 mg of a more polar product (Rf=0.30, 1:1 EtOAc/Hexane). Analytical HPLC of this band revealed a 1:3 ratio of isomeric cyclized products. More polar acylenamine (6) (600 mg) was cyclized as described above and gave 300 mg of product mixture which was identical in composition to that derived from acylenamine (5). The combined products were separated by HPLC (35% EtOAc/Hexane). The less polar component (125 mg) gave NMR ($CDCl_3$); 7.60 (m,4H); 7.0-7.3 (m,4H); 5.34 (m,2H); 4.86 (dd, 1H, Ja=5 $H_z$, Jb=6 $H_z$); 3.60 (S,3H); 3.37 (m,2H); 2.0-2.7 (m,4H): The more polar component (8) (400 mg) gave NMR ($CDCl_3$); 7.60 (M,4H); 7.0-7.3 (m,4H); 5.34 (m,2H); 4.60 (dd, 1H, Ja=6 H $_z$, Jb=5 $H_z$); 3.70 (s,3H); 3.40 (m,2H); 2.0-2.7 (m,4H).

It was deduced that these isomeric cyclized products were isomeric about the carbon bearing the terminal carbomethoxyl group.

EXAMPLE 5

6-amino-2,3,5,6,7,11b-hexahydro-5-oxo-1H-pyrrole[2,1-a]-[2]benzaepine-3-carboxylic acid (mixture of isomers)

A stirred solution of 101 mg (0.25 mM) phthalimide (7a) in 1 mL $CH_3OH$ was treated with 0.5 mL 1N $H_2NNH_2 \bullet H_2O$ in $CH_3OH$ at 25°C under $N_2$ for 3 days. The solution was diluted with $CHCl_3$ and filtered. The filtrate was concentrated, redissolved in 2 mL $CH_3OH$, and treated with 0.5 mL 1N LiOH. After stirring for 10 hours, the solution was concentrated and the residue was dissolved in 2 mL $H_2O$ and acidified with 0.5 mL 1N HCl solution. Filtration gave 55 mg (0.21 mM) of zwitterion (8a): NMR ($CD_3CN$,TFA); 7.0-7.3 (m,4H); 4.80 (dd, 1H, Ja=6 $H_z$, Jb=3 $H_z$); 4.50 (dd, 1H, Ja=8 $H_z$, Jb=4 $H_z$); 4.05 (dd, 1H, Ja=9 $H_z$ , Jb=6 $H_z$), 3.43 (dd, 1H, Ja=17 $H_z$, Jb=6 $H_z$); 2.85 (dd, 1H, Ja=17 $H_z$, Jb=9 $H_z$ ); 2.0-2.7 (m,4H).

Isomeric protected dipeptide (7b) 300 mg was deprotected in a similar manner to give 156 mg of zwitterion (8b) which had NMR spectra nearly identical to that of (8a).

EXAMPLE 6

(3 ,6 )-6-[[1-(ethoxycarbonyl)-3-phenylpropyl]amino]-2,3,5,6,7,11b-hexahydro-5-oxo-1H-pyrrolo[2,1-a][2]-benzazepine-3-carboxylic acid

To a stirred solution of 50 mg (0.20 mM) of zwitterion (8a) and 88 mg (0.40 mM) of "proton sponge" in 1.0 mL of dry DMF was added 70 mg (0.21 mM) of R-triflate (9) and the resulting solution was stirred at 25°C for 8 hours. The solution was evaporated to dryness under reduced pressure. The residue was dissolved in 2 mL $H_2O$, triturated with ether, and acidified with 0.20 mL 1N HCl. The coupled zwitterion was isolated by filtration to give 65 mg of a colorless powder: NMR ($CD_3CN$,TFA); 7.15 (m,9H); 5.39 (d,1H,J=8 $H_z$); 4.86 (dd, 1H, $J_a$=11 $H_z$, Jb=6 $H_z$); 4.48 (dd, 1H, Ja=9 $H_z$, Jb=2 $H_z$); 4.15 (q, 2H, J=7 $H_z$); 4.15 (t, 1H, J=6 $H_z$); 3.64 (dd, 1H, Ja=16 $H_z$, Jb=6 $H_z$ ); 3.34 (dd, 1H, Ja=16 $H_z$, Jb=11 $H_z$); 2.85 (m,2H); 2.35 (m,2H); 1.30 (t, 3H, J=7 $H_z$).

EXAMPLE 7

(3 , 6)-6-[[1-(ethoxycarbonyl)-3-phenylpropyl]amino]-2,3,5,6,7,11b-hexahydro-5-oxo-1H-pyrrolo[2,1-a][2]-benzazepine-3-carboxylic acid

To a stirred solution of 150 mg (0.60 mM) of zwitterion (8b) and 264 mg (1.20 mM) of "proton sponge" in 1.0 mL of dry DMF was added 210 mg (0.63 mM) of R-triflate (9) and the resulting solution was stirred at 25°C under $N_2$ for 8 hours. The solution was evaporated to dryness under reduced pressure. The residue was dissolved in 2 mL $H_2O$, triturated with ether, and acidified with 0.60 mL 1N HCl. The coupled zwitterion was collected by filtration to give 195 mg of a white powder: NMR ($CD_3CN$,TFA); 7.15 (m,9H); 5.46 (d, 1H, J=8 $H_z$); 4.92 (dd, 1H, Ja=9 $H_z$, Jb=2 $H_z$); 4.48 (dd, 1H, Ja=9 $H_z$, Jb=3 $H_z$); 4.08 (t, 1H, J=6 $H_z$ ); 3.69 (dd, 1H, Ja=16 $H_z$, Jb=6 $H_z$); 3.35 (dd, 1H, Ja=16 $H_z$, Jb=11 $H_z$); 2.85 (m,2H); 2.35 (m,2H); 1.30 (t,3H, J=7 $H_z$).

Prodrug 10a was the more potent.

The compounds of this invention form salts with various inorganic and organic acids and bases which are also within the scope of the invention. Such salts include ammonium salts, alkali metal salts like sodium and potassium salts, alkaline earth metal salts like the calcium and magnesium salts, salts with organic bases e.g., dicyclohexylamine salts, N-methyl-D-glucamine, salts with amino acids like arginine, lysine and the like. Also salts with organic and inorganic acids can be prepared, e.g., HCl, HBr, $H_2CO_3$, $H_3PO_4$, methanesulfonic, toluenesulfonic, maleic, fumaric, camphorsulfonic. The non-toxic physiologically acceptable salts are preferred, although other salts are also useful, e.g., in isolating or purifying the product.

The salts can be formed by conventional means as by reacting the free acid or free base forms of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

The compounds of this invention inhibit angiotensin converting enzyme and thus block conversion of the decapeptide angiotensin I to angiotensin II. Angiotensin II is a potent pressor substance. Thus, blood-pressure lowering can result from inhibition of its biosynthesis especially in animals and humans whose hypertension is angiotensin II related. Furthermore, converting enzyme may lower blood-pressure also by potentiation of bradykinin. Although the relative importance of these and other possible mechanisms remains to be established, inhibitors of angiotensin converting enzyme are effective antihypertensive agents in a variety of animal models and are useful clinically, for example, in many human patients with renovascular, malignant and essential hypertension. See, for example, D. W. Cushman et al., Biochemistry 16, 5484 (1977).

The evaluation of converting enzyme inhibitors is guided by in vitro enzyme inhibition assays. For example, a useful method is that of Y. Piquilloud, A. Reinharz and M. Roth, Biochem. Biophys. Acta, 206 N36 (1970) in which the hydrolysis of carbobenzyloxyyphenylalanylhistidinylleucine is measured. In vivo evaluations may be made, for example, in normotensive rats challenged with angiotensin I by the technique of J. R. Weeks and J. A. Jones, Proc. Soc. Exp. Biol. Med., 104, 646 (1960) or in a high renin rat model such as that of S. Koletsky et al., Proc. Soc. Exp. Biol. Med. 125, 96 (1967).

Thus, the compounds of this invention are useful as antihypertensives in treating hypertensive mammals, including humans, and they can be utilized to achieve the reduction of blood pressure by formulating them in appropriate compositions for administration. The compounds of this invention can be administered to patients in need of such treatment in a dosage range of 0.5 to 100 mg per patient generally given several times a day, thus giving a total daily dose of from 0.5 to 400 mg per day. The dose will vary depending on severity of disease, weight of patient and other factors which a person skilled in the art will recognize.

Thus, in accordance with the present invention there is provided a pharmaceutical composition for inhibiting angiotensin converting enzyme or treating hypertension comprising a pharmaceutically acceptable carrier and a pharmaceutically effective amount of a compound of Formula I.

For administration, the compositions of the invention can also contain other conventional pharmaceutically acceptable compounding ingredients, as necessary or desired. Such ingredients are generally referred to as carriers or diluents. Conventional procedures for preparing such compositions in appropriate dosage forms can be utilized. Whatever the dosage form, it will contain a pharmaceutically effective amount of the compound of the invention.

The present compositions can be administered orally or other than orally, e.g., parenterally, by insufflation, topically, rectally, etc.; using appropriate dosage forms; e.g., tablets, capsules, suspensions, solutions, and the like, for oral administration; suspension emulsions, and the like, for parenteral administration; solutions for intravenous administration; the ointments, transdermal patches, and the like, for topical administration.

Compositions intended for oral use may be prepared according to any method known to the art for the manufacture of pharmaceutical compositions and such compositions may contain one or more agents such as sweetening agents, flavoring agents, coloring agents and preserving agents in order to provide pharmaceutically elegant and palatable preparation. Tablets containing the active ingredient in admixture with non-toxic pharmaceutically acceptable excipients may also be manufactured by known methods. The excipients used may be for example, (1) inert diluents such as calcium carbonate, sodium carbonate, lactose, calcium phosphate or sodium phosphate; (2) granulating and disintegrating agents such as corn starch, or alginic acid (3) binding agents such as starch, or gelatin or acacia, and (4) lubricating agents such as magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Pat. No. 4,256,108; 4,160,452; and 4,265,874 to form osmotic therapeutic tablets for controlled release.

In some cases, formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, for example, peanut oil, liquid paraffin, or olive oil.

Aqueous suspensions normally contain the active materials in admixture with excipients suitable for the manufacture of aqueous suspensions. Such excipients may be

(1) suspending agents such as sodium carboxymethylcellulose, methylcellulose, hydroxypropylmethylcellulose, sodium alginate, polyvinylpyrrolidone, gum tragacanth and gum acacia;

(2) dispersing or wetting agents which may

(a) a naturally-occuring phosphatide such as lecithin,

(b) a condensation product of an alkylene oxide with a fatty acid, for example, polyoxyethylene stearate,

(c) a condensation product of ethylene oxide with a long chain aliphatic alcohol, for example, heptadecaethyleneoxycetanol,

(d) a condensation product of ethylene oxide with a partial ester derived from a fatty acid and a hexitol such as polyoxyethylene sorbitol monooleate, or

(e) a condensation product of an ethylene oxide with a partial ester derived from a fatty acid and a hexitol anhydride, for example, polyoxyethylene sorbitan monooleate.

The aqueous suspensions may also contain one or more preservatives, for example, ethyl or n-propyl p-hydroxybenzoate; one or more coloring agents; one or more flavoring agents; and one or more sweetening agents such as sucrose or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide a palatable oral preparation. These compositions may be preserved by the addition of an antioxidant such as ascorbic acid.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example, those sweetening, flavoring and coloring agents described above may also be present.

The pharmaceutical compositions of the invention may also be in the form of oil-in-water emulsions. The oily phase may be vegetable oil such as liquid paraffin or a mixture thereof. Suitable emulsifying agents may be (1) naturally-occurring gums such as gum acacia and gum tragacanth, (2) naturally-occuring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Syrups and elixirs may be formulated with sweetening agents, for example, glycerol, propylene glycol, sorbitol or sucrose. Such formulations may also contain a demulcent, a preservative and flavoring and coloring agents.

The pharmaceutical compositions may be in the form of a sterile injectable aqueous or oleagenous suspension. This suspension may be formulated according to known methods using those suitable dispersing or wetting agents and suspending agents which have been mentioned above. The sterile injectable preparation may also be a sterile injectable solution or suspension in a non-toxic parenterally-acceptable diluent or solvent, for example vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

A composition of the invention may also be administered in the form of suppositories for rectal administration of the drug. These compositions can be prepared by mixing the drug with a suitable nonirritating excipient which is solid at ordinary temperatures by liquid at the rectal temperature and will therefore melt in the rectum to release the drug. Such materials are cocoa butter and polyethylene glycols.

For topical use, creams, ointments, jellies, solutions or suspensions, etc., containing the compositions of the invention are employed.

The amount of active ingredient that may be combined with the carrier materials to produce a single dosage form will vary depending upon the host treated and the particular mode of administration. For example, a formulation intended for oral administration may contain from 5 mg to 5 g of active agent compounded with an appropriate and convenient amount of carrier material which may vary from about 5 to 95 percent of the total composition.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The compounds of this invention can also be administered in combination with other antihypertensives and/or diuretics and/or calcium entry blockers. For example, the compounds of this invention can be given in combination with such compounds as acetazolamide, amiloride, aminophylline, atenolol,bendroflumethiazide, benzthiazide, bumetanide, chlorothalidone, chlorothiazide, clonidine, cryptenamine acetates and cryptenamine tannates, cyclothiazide, deserpidine, diazoxide, diltiazem, (S)-1-[[2-(3,4-dimethoxyphenyl)-ethyl]amino]-3-[4-(2-thienyl)-1H-imidazol-2-yl]phenoxy]-2-propanol, thacrynic acid, flumethiazide, furosemide, guanethidene sulfate, hydralazine hydrochloride, hydrochlorothiazide, hydroflumethiazide, (+)-4-[3-[-[2-(1-hydroxycyclohexyl) ethyl]-4-oxo-2-thiazolidinyl]-propyl]-benzoic acid, indacrinone and variable ratios of its enantiomers, merethoxylline procaine, methylclothiazide, methyldopa, methyldopate hydrochloride, metolazone, metoprolol tartate, minoxidil, naldolol, pargyline hydrochloride, pindolol, polythiazide, prazosin, propranolol, quinethazone, rauwolfia serpentina, rescinnamine, reserpine, sodium ethacrynate, sodium nitroprusside, spironolactone, ticrynafen, timolol, triamterene, trichlormethiazide, trimethophan camsylate, bepridil, diltiazim, etafenone, falipamil, felodipine, flunarizine, gallopamil, indapamide, lidoflazine, nicardipine, nifedipine, nimopidine, nitrendipine, perhexiline, prenylamine, tiapamil, verapamil, and the like, as well as admixtures and combinations thereof.

Typically, the individual daily dosages for these combinations can range from about one fifth of the minimally recommended clinical dosages to the maximum recommended levels for the entities when they are given singly.

To illustrate these combinations, one of the antihypertensives of this invention effective in the 0.5 to 1000 mg per day range can be effectively combined with the following compounds at the indicated per day dose range: hydrochlorothiazide (10-100 mg); chlorothiazide (125-2000 mg); manipulated indacrinone enantiomer ratio (25-150 mg); ethacrynic acid (15-2000 mg); amiloride (5-20 mg); furosemide (5-80 mg); propranolol (20-480 mg); timolol (5-60 mg); and methyldopa (65-2000 mg); and the pivaloyloxyethyl ester of methyldopa (30-1000 mg). In addition, triple drug combinations of hydrochlorothiazide (10-100 mg) plus amiloride (5-20 mg) plus converting enzyme inhibitor of this invention (0.5-1000 mg) or manipulated indacrinone enantiomer ratio (25-150 mg) plus amiloride (5-20 mg) plus converting enzyme inhibitor of this invention (0.5-1000 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

## Claims

CLAIMS FOR THE FOLOWING CONTRATING STATES: BE CH DE FR GB IT LI LU NL SE

1. A compound of the formula

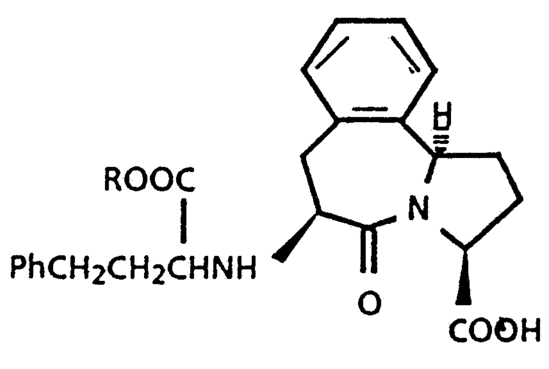

or a pharmaceutically acceptable salt thereof, wherein R is a $C_{1-6}$ alkyl and Ph is phenyl.

2. A compound of claim 1 wherein R is ethyl.

3. Compounds of claim 1 wherein they are in admixture with their diastereomeric isomers.

4. A process for preparing a compound of claim 1 which comprises coupling a fused lactam of the formula

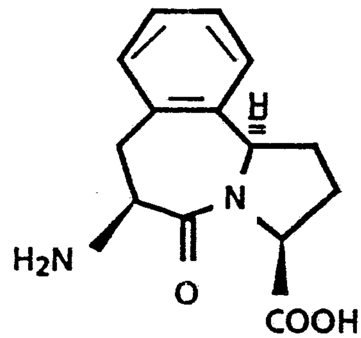

with an R-triflate of the formula

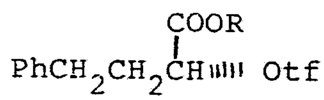

, with Tf being trifluoromethanesulfonyl, in the presence of a base.

5. A compound of claim 1, 2 or 3 for use as a medicine.

6. Use of a compound of claim 1, 2 or 3 for preparing a medicament for treating hypertension.

7. A pharmaceutical composition which comprises a compound of claim 1, 2 or 3 in admixture with a pharmaceutically acceptable carrier.

8. A medicament which comprises a compound of claim 1, 2 or 3 for conjunctive administration with another antihypertensive and/or diuretic and/or calcium entry blocker.

9. A pharmaceutical composition which comprises a compound of claim 1, 2 or 3 and one or two other active ingredients selected from another antihypertensive, a diuretic and a calcium entry blocker.

CLAIMS FOR THE FOLLOWING CONTRACTING STATES: AT ES GR

1. A process for preparing a compound of the formula

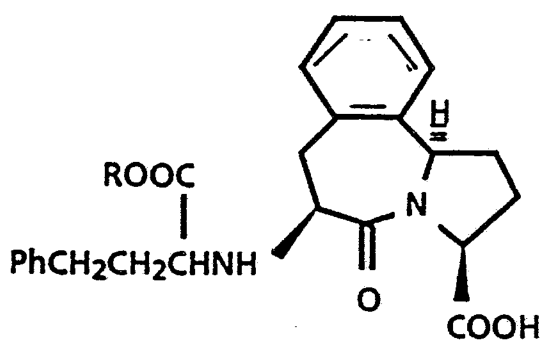

or a pharmaceutically acceptable salt thereof, wherein R is a $C_{1-6}$ alkyl and Ph is phenyl, which comprises coupling a fused lactam of the formula

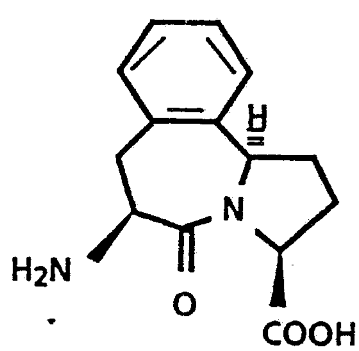

with an R-triflate of the formula

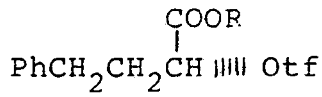

, with Tf being trifluoromethanesulfonyl, said coupling being effected by contacting said reactants in the presence of a base.

2. A process according to claim 1 for preparing a compound wherein R is ethyl.

3. Use of a compound of claim 1 or 2 for preparing a medicament for treating hypertension.